(19) Europäisches Patentamt
European Patent Office
Office européen des brevets

(11) Numéro de publication : **0 300 865 B1**

(12) # FASCICULE DE BREVET EUROPEEN

(45) Date de publication du fascicule du brevet : **18.03.92 Bulletin 92/12**

(21) Numéro de dépôt : **88401741.9**

(22) Date de dépôt : **05.07.88**

(51) Int. Cl.$^5$ : **C07C 233/77,** C07D 295/125, C07D 213/38, C07D 215/50, C07D 213/81, C07C 255/50, C07D 217/26, A61K 31/165, A61K 31/395

(54) **Dérivés de N-aminobutyl N-phényl arylamides, leur préparation et leur application en thérapeutique.**

Le dossier contient des informations techniques présentées postérieurement au dépôt de la demande et ne figurant pas dans le présent fascicule.

(30) Priorité : **16.07.87 FR 8710026**

(43) Date de publication de la demande : **25.01.89 Bulletin 89/04**

(45) Mention de la délivrance du brevet : **18.03.92 Bulletin 92/12**

(84) Etats contractants désignés : **AT BE CH DE ES FR GB GR IT LI LU NL SE**

(56) Documents cités :
**GB-A- 1 232 787**
**GB-A- 1 282 600**

(56) Documents cités :
**CHEMICAL ABSTRACTS, vol. 103, no. 1, 8 juillet 1985, page 538, résumé no. 5978e, Columbus, Ohio, US; X. ZHOU et al.: "Synthesis of benzanilide derivatives and their antiarrhythmic effects"**
**CHEMICAL ABSTRACTS, vol. 82, no. 1, 6 janvier 1975, pages 366-367, résumé no. 4223r, Columbus, Ohio, US; K. NAGARAJAN et al.: "Condensed heterotricycles. Amino and aminoalkydibenz[b,f][1,4]oxazepin-11(10H)-ones"**

(73) Titulaire : **SYNTHELABO**
**58 rue de la Glacière**
**F-75013 Paris (FR)**

(72) Inventeur : **Hoornaert, Christian**
**103, rue Didot**
**F-75014 Paris (FR)**
Inventeur : **Muller, Jean-Claude**
**4, Avenue de l'Espérance**
**F-91390 Morsang S/Orge (FR)**
Inventeur : **Beeley, Nigel**
**16 Cheshire Road**
**Thames Oxfordshire OX 9 3 LQ (GB)**

(74) Mandataire : **Ludwig, Jacques et al**
**SYNTHELABO, Service Brevets, 22, avenue Galilée, B.P. 72**
**F-92352 Le Plessis Robinson Cédex (FR)**

EP 0 300 865 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés de N-aminobutyl N-phényl arylamides, leur préparation et leur application en thérapeutique.

Les composés de l'invention répondent à la formule générale (I) donnée dans le schéma 1 ci-après, formule dans laquelle

X représente un atome d'hydrogène ou d'halogène ou un groupe trifluorométhyle, alkyle en $C_1$-$C_4$ ou alcoxy en $C_1$-$C_4$,

$R_1$ représente un groupe alkyle droit ou ramifié en $C_2$-$C_8$, un groupe cycloalkyle en $C_3$-$C_5$ ou un groupe cycloalkylméthyle dont le cycle est en $C_3$-$C_5$,

$R_2$, pris isolément, représente un atome d'hydrogène ou un groupe alkyle en $C_1$-$C_4$,

$R_3$, pris isolément, représente un atome d'hydrogène ou un groupe alkyle en $C_1$-$C_4$, un groupe phénylalkyle éventuellement substitué ou un groupe pyridinylalkyle, ou bien,

$R_2$ et $R_3$, pris ensemble, représentent avec l'atome d'azote qui les porte un cycle pyrrolidinyle, pipéridinyle, morpholinyle, perhydrothiazinyle, pipérazinyle ou méthyl-4 pipérazinyle, et

Ar représente

- soit un groupe phényle portant éventuellement de un à trois substituants choisis parmi les atomes d'halogène, les groupes alkyle en $C_1$-$C_4$, alcoxy en $C_1$-$C_4$, trifluorométhyle, nitro et cyano,
- soit un groupe naphtyle, pyridinyle, quinoléinyle ou isoquinoléinyle.

Les composés préférés sont ceux dans la formule (I) desquels $R_1$ représente un groupe isobutyle et Ar représente un groupe phényle substitué, en particulier lorsque X représente un atome d'hydrogène et $R_2$ représente un groupe méthyle. Parmi ces derniers on distingue spécialement les composés dans la formule desquels $R_3$ représente un groupe méthyle et Ar représente un groupe diméthyl-2,3 phényle, trifluorométhyl-3 phényle, nitro-3 phényle ou méthoxy-2 nitro-5 phényle ; ceux dans la formule desquels $R_3$ représente un groupe (méthoxy-3 phényl)-2 éthyle, (diméthoxy-3,4 phényl)-2 éthyle ou (pyridinyl-3)-2 éthyle et Ar représente un groupe trifluorométhyl-3 phényle ; et celui dans la formule duquel $R_3$ représente un groupe (diméthoxy-3,4 phényl)-2 éthyle et Ar représente un groupe méthyl-2 nitro-3 phényle.

Les composés de l'invention peuvent se présenter à l'état de bases libres ou de sels d'addition à des acides acceptables en pharmacologie.

Des composés de structures chimiques analogues à celle des composés de l'invention sont décrits dans les brevets GB-1232787 et GB-1282600, et dans Shanghai Diyi Yixueyuan Xuebo 11(6), 412-417 (1984).

Conformément à l'invention, on peut préparer les composés de formule (I) par une acylation selon le schéma 1 ci-après. On fait réagir une diamine de formule (II), dans laquelle X, $R_1$, $R_2$ et $R_3$ sont tels que définis ci-dessus, avec un chlorure d'acide de formule (III) dans laquelle Ar est tel que défini ci-dessus, à froid, dans un solvant et en présence d'une base telle que le carbonate de potassium.

La diamine de formule (II) peut être obtenue par exemple selon l'un des deux procédés illustrés par les schémas 1a et 1b ci-après.

Selon le schéma la, le composé de départ est une benzènamine primaire de formule (IV), dans laquelle X et $R_1$ sont tels que définis ci-dessus. On effectue d'abord une acylation au moyen d'un chlorure de chloro-4 butanoyle de formule (V), à froid et en présence d'une base. On fait ensuite réagir l'amide de formule (VI) ainsi obtenu avec une amine de formule (VII), dans laquelle $R_2$ et $R_3$ sont tels que définis ci-dessus, dans un solvant tel que l'acétonitrile, le diméthylformamide, l'éthanol ou le tétrahydrofuranne, à chaud, en présence d'iodure de potassium, d'un agent de transfert de phase tel que l'iodure de tétrabutylammonium, et d'une base telle que le carbonate de potassium.

Enfin ou soumet l'amide de formule (VIII) ainsi obtenu à une réduction, par exemple au moyen d'hydrure d'aluminium et lithium, ou de diborane, ou de borohydrure de métal alcalin, dans un solvant à chaud.

Selon le schéma 1b, la diamine de formule (II) peut également être préparée à partir de l'acétamide de formule (IX), dans laquelle X et $R_1$ sont tels que définis ci-dessus. L'acétamide de formule (IX) lui-même peut être préparé de manière connue à partir de la benzènamine de formule (IV).

On fait réagir l'acétamide de formule (IX) avec le bromo-1 chloro-4 butane de formule (X), à froid et dans un solvant polaire aprotique tel que le diméthylformamide, le diméthylsulfoxyde ou l'acétone, en présence d'une base telle que l'hydrure de sodium, la potasse ou le carbonate de potassium.

On traite ensuite le composé de formule (XI) ainsi obtenu avec une amine de formule (VII), de la manière indiquée ci-dessus à propos de l'amide de formule (VI).

On obtient ainsi un composé de formule (XII) que l'on peut transformer en diamine de formule (II) par exemple en présence d'eau et d'une base telle que le tert-butylate de potassium ou la potasse, à chaud et dans un solvant tel que le tétrahydrofuranne ou le dioxanne.

Un autre procédé de préparation des composés de formule (I) est illustré par le schéma 2 ci-après.

Le composé de départ est une benzènamine de formule (IV), dans laquelle X et $R_1$ sont tels définis ci-dessus. On le soumet à une acylation par un chlorure d'acide de formule (III), dans laquelle Ar est telle que défini ci-dessus, entre -10 et +30°C, dans un solvant tel que le dichlorométhane, l'éther ou le tétrahydrofuranne, et en présence d'une base telle que le carbonate de potassium, la triéthylamine ou la (diméthylamino)-4 pyridine. On obtient ainsi un amide de formule (XIII) que l'on fait réagir avec le bromo-1 chloro-4 butane de formule (X), de la manière indiquée ci-dessus à propos de l'amide de formule (IX).

Enfin on traite le composé de formule (XIV) ainsi obtenu avec une amine de formule (VIII), de la manière indiquée ci-dessus à propos de l'amide de formule (VI).

La benzènamine de formule (IV), qui est à l'origine de toutes les variantes des procédés de l'invention, peut être obtenue selon diverses méthodes connues.

On peut par exemple alkyler un N-(hydroxy-2 phényl)acétamide portant le substituant X pour obtenir le N-(alcoxy-2 phényl)acétamide correspondant, puis le dégrader en benzènamine de formule (IV) par hydrolyse.

On peut aussi partir d'un halogéno-2 nitrobenzène portant le substituant X, et le faire réagir avec un alcool de formule $R_1OH$, pour obtenir l'alcoxy-2 nitrobenzène correspondant, puis réduire ce dernier en benzènamine de formule (IV).

Enfin on peut partir d'un nitro-2 phénol portant le substituant X, l'alkyler pour obtenir l'alcoxy-2 nitrobenzène correspondant, et réduire ce dernier en benzènamine de formule (IV).

Schéma 1

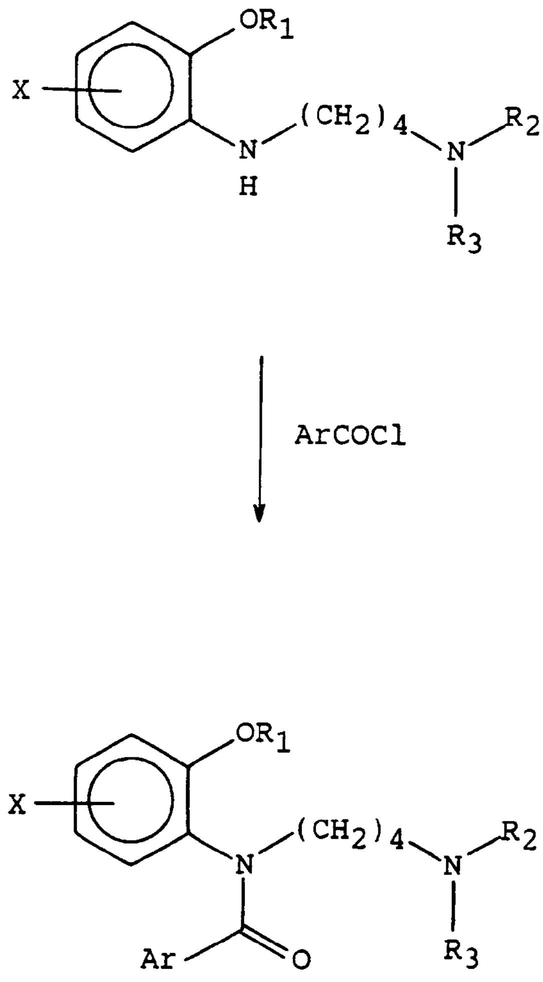


(II)

(III)

(I)

Schéma 1a

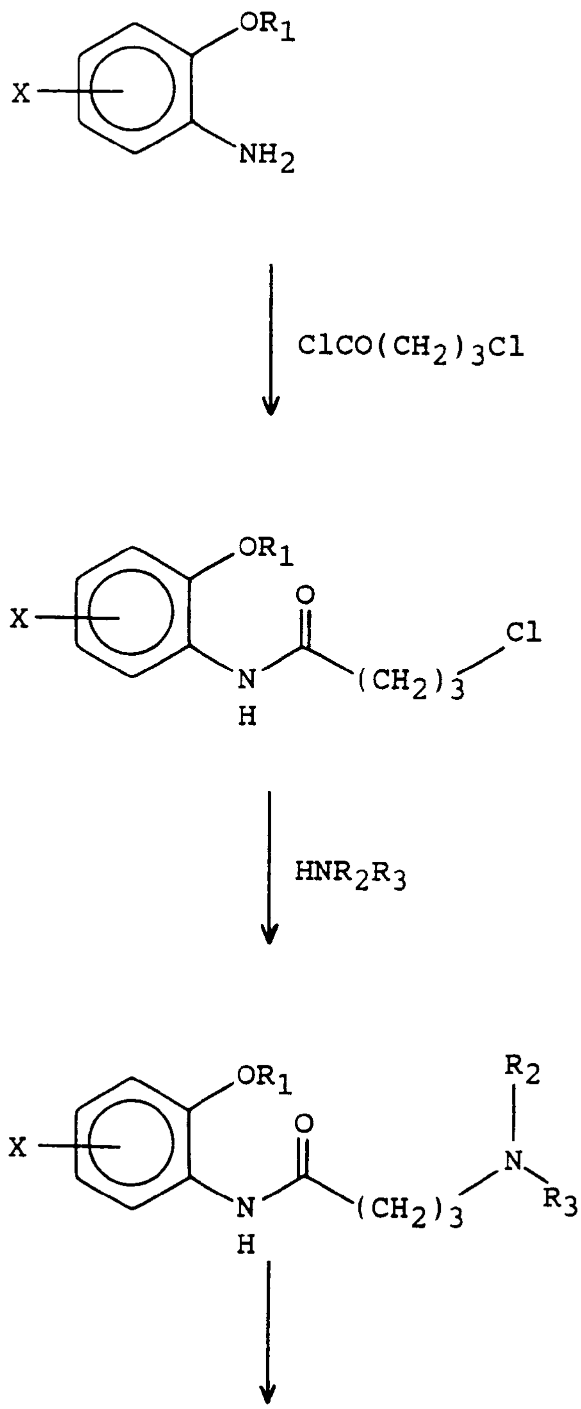


(IV)

(V)

(VI)

(VII)

(VIII)

(II)

Schéma 1b

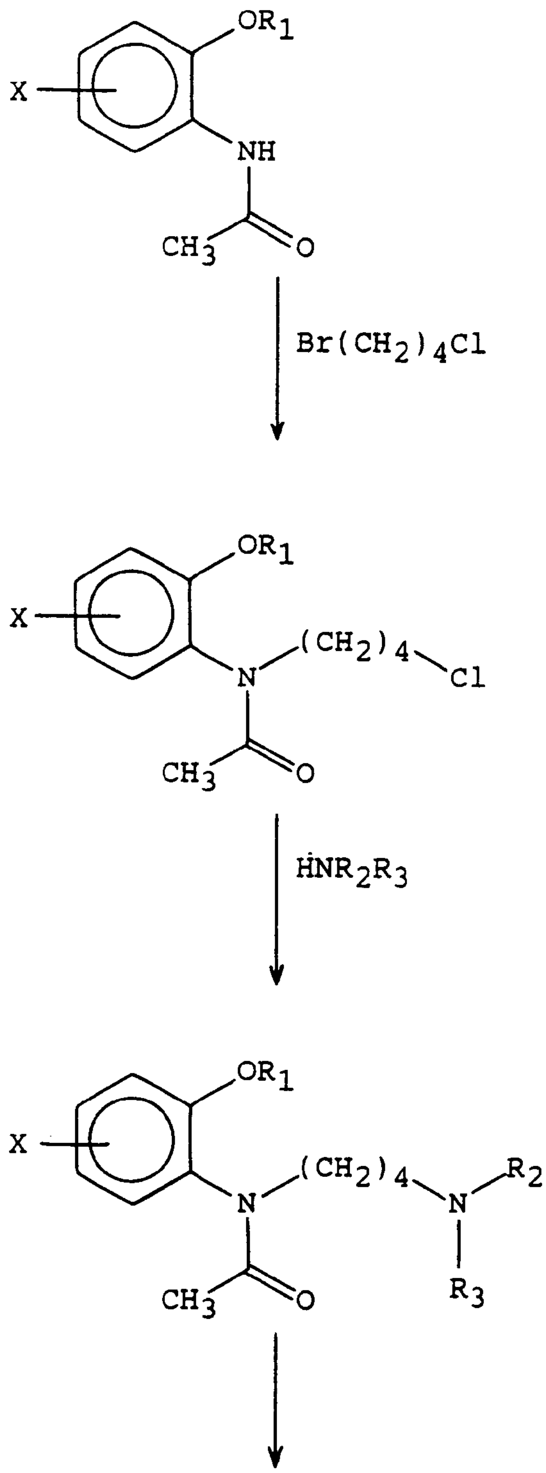


(IX)

(X)

(XI)

(VII)

(XII)

(II)

Schéma 2

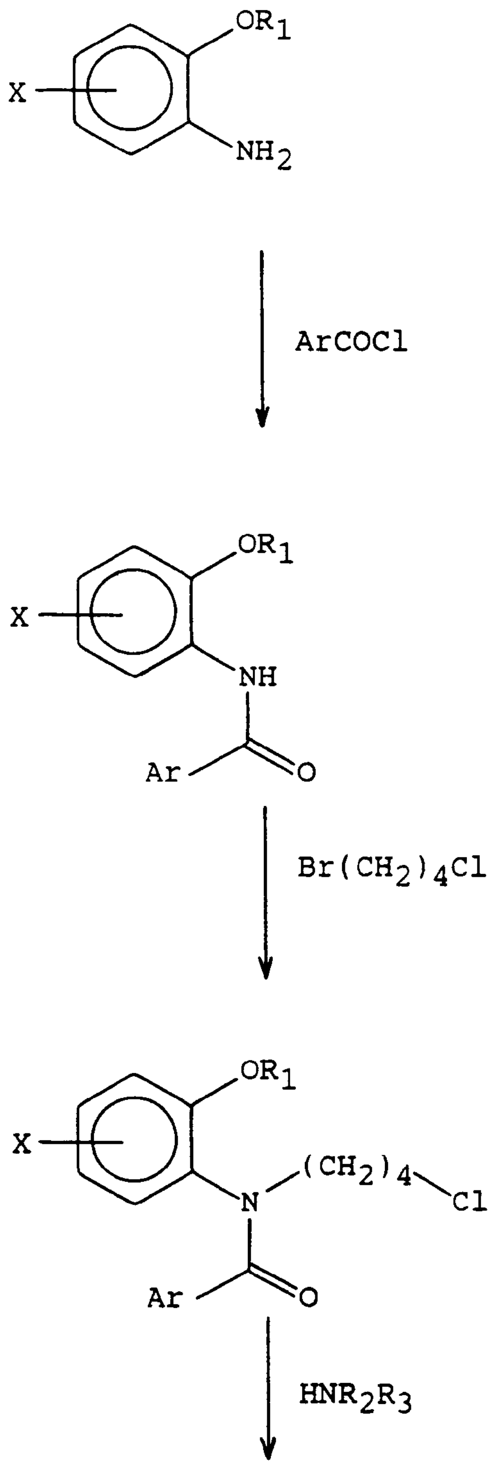


(IV)

(III)

(XIII)

(X)

(XIV)

(VII)

(I)

Les exemples qui vont suivre illustrent en détail la préparation de quelques composés selon l'invention. Les microanalyses et les spectres IR et RMN ont confirmé les structures des produits obtenus.
Les numéros indiqués entre parenthèses dans les titres des exemples correspondent à ceux du tableau donné plus loin.

Exemple 1. (Composé N°23)

N-[(diméthylamino)-4 butyl] N-[(méthyl-2 propoxy)-2 phényl] dichloro-2,5 benzamide, fumarate.

a) Chloro-4 N-[(méthyl-2 propoxy)-2 phényl]-butanamide.

On ajoute goutte à goutte une solution de 44 ml de chlorure de chloro-4 butyryle dans 350 ml d'éther à une suspension de 60 g de (méthyl-2 propoxy)-2 benzènamine et 199 g de carbonate de potassium dans 500 ml d'éther, refroidie par un bain de glace, et on agite le mélange pendant une nuit à température ambiante. On filtre le mélange, et on lave le filtrat successivement avec 200 ml de soude 1N, trois fois 100 ml d'eau et 100 ml de solution saturée de chlorure de sodium. On sèche la solution éthérée sur sulfate de magnésium et on évapore le solvant. On recueille 85,8 g de produit sirupeux qu'on utilise tel quel dans l'étape suivante.

b) (Diméthylamino)-4 N-[(méthyl-2 propoxy)-2 phényl]-butanamide.

On dissout le produit précédent dans 620 ml d'acétonitrile et on ajoute 126 g de chlorhydrate de diméthylamine, 51,5 g d'iodure de potassium, 107 g de carbonate de potassium et 5,7 g d'iodure de tétra-n-butylammonium. On chauffe le mélange au reflux pendant 7 h, on le filtre en lavant le solide avec de l'acétonitrile, et on évapore le filtrat.

On reprend le résidu avec 1000 ml d'éther et 500 ml de soude 1N. On sépare la phase organique, on la lave avec une solution saturée de chlorure de sodium, on la sèche sur sulfate de magnésium et on l'évapore sous vide. On obtient 45 g de produit brut.

En extrayant les phases aqueuses trois fois avec 200 ml de dichlorométhane on obtient, après séchage et évaporation, encore 19 g de produit qu'on ajoute aux 45 g déjà isolés. On purifie le tout par formation du chlorhydrate correspondant et recristallisation. Après libération de la base par traitement basique, on recueille 52 g de produit sirupeux qu'on utilise tel quel dans l'étape suivante.

c) N,N-diméthyl N'-[(méthyl-2 propoxy)-2 phényl]-butanediamine-1,4.

On dissout 52 g du produit précédent dans 300 ml de tétrahydrofuranne et on ajoute la solution obtenue, goutte à goutte, à une suspension de 14 g d'hydrure d'aluminium et lithium dans 600 ml de tétrahydrofuranne refroidie par un bain de glace.

Puis on chauffe le mélange au reflux pendant 3h, on laisse refroidir et on le traite avec une solution de 19 g de soude dans 25 ml d'au.

On dilue le mélange avec 500 ml d'acétate d'éthyle et on le filtre sur sulfate de magnésium. On évapore le filtrat et on distille le résidu sous vide, ce qui donne 31,3 g de produit. Point d'ébullition : 118-124°C à 20 Pa (0,15 mmHg).

d) N-[(diméthylamino)-4 butyl] N-[(méthyl-2 propoxy)-2 phényl] dichloro-2,5 benzamide.

On ajoute goutte à goutte une solution de 2,3 g de chlorure de dichloro-2,5 benzoyle dans 30 ml d'éther à un mélange de 2,4 g du composé précédent, 4 g de carbonate de potassium et 30 ml d'éther.

On agite le mélange pendant une nuit, puis on ajoute 100 ml d'eau et 40 ml d'éther. On sépare la phase organique, on la lave trois fois avec 50 ml d'eau puis avec 50 ml d'une solution saturée de chlorure de sodium, on la sèche sur sulfate de magnésium et on l'évapore sous vide. Après purification par chromatographie sur colonne de silice on obtient 3,2 g de produit sirupeux.

On en dissout 3 g dans du méthanol, on ajoute 0,8 g d'acide fumarique, on chasse le solvant sous vide et on recristallise le résidu dans l'acétate d'éthyle. On obtient 2,9 g de fumarate sous forme de poudre blanche. Point de fusion : 130°C.

Exemple 2. (Composé N°2)

N-[(diméthylamino)-4 butyl] N-[(méthyl-2 propoxy)-2 phényl] (trifluorométhyl)-3 benzamide, chlorhydrate.

a) N-(chloro-4 butyl) N-[(méthyl-2 propoxy)-2 phényl]acétamide.

A une suspension de 10,3 g d'hydrure de lithium (à 50 % dans l'huile) dans 100 ml de diméthylformamide, refroidie par un bain de glace, on ajoute goutte à goutte 35,6 g de N-[(méthyl-2 propoxy)-2 phényl]acétamide en solution dans 70 ml de diméthylformamide, puis 21,5 ml de bromo-1 chloro-4 butane.

On agite le mélange pendant 5 h à température ambiante ; on ajoute 400 ml d'eau et 400 ml d'éther, on sépare la phase organique, on la lave trois fois avec 50 ml d'eau puis avec 25 ml d'une solution saturée de chlorure de sodium, on la sèche sur sulfate de magnésium et on l'évapore sous vide. On obtient 54,5 g de produit sirupeux qu'on utilise tel quel dans l'étape suivante.

b) N-[(diméthylamino)-4 butyl] N-[(méthyl-2 propoxy)-2 phényl]acétamide.

On dissout le produit précédent dans 120 ml de diméthylformamide on ajoute 117 g de carbonate de potassium, 20 g d'iodure de potassium, 3 g d'iodure de tétra-n-butylammonium et 69 g de chlorhydrate de diméthylamine.

On agite le mélange pendant 13 h à 70°C, on le filtre, en lavant le solide trois fois avec 200 ml d'éther ; on traite le filtrat avec 400 ml de soude 0,1N, on sépare la phase organique, on la lave trois fois avec 100 ml d'eau puis avec 50 ml d'une solution saturée de chlorure de sodium, on la sèche sur sulfate de magnésium et on l'évapore sous vide.

On obtient 42,1 g de produit sirupeux qu'on utilise tel quel dans l'étape suivante.

c) N,N-diméthyl N′-[(méthyl-2 propoxy)-2 phényl]-butanediamine-1,4.

On dissout le produit précédent dans 280 ml de tétrahydrofuranne, on ajoute 4,9 ml d'eau et 92,4 g de tert-butylate de potassium, et on chauffe le mélange au reflux pendant 12 h. On ajoute 200 ml d'eau glacée et 600 ml d'éther, on sépare la phase organique, on la lave quatre fois avec 100 ml d'eau puis avec 50 ml d'une solution saturée de chlorure de sodium, on la sèche sur sulfate de magnésium et on l'évapore sous vide. Après distillation sous vide du résidu on obtient 27,1 g de produit.

Point d'ébullition : 106-110°C à 13 Pa (0,1 mmHg).

d) N-[(diméthylamino)-4 butyl] N-[(méthyl-2 propoxy)-2 phényl] (trifluorométhyl)-3 benzamide.

On prépare une suspension de 5,0 g du produit précédent et 10,1 g de carbonate de potassium dans 60 ml de dichlorométhane, on la refroidit par un bain de glace, et on ajoute une solution de 3,4 ml de chlorure de (trifluorométhyl)-3 benzoyle dans 60 ml de dichlorométhane.

On agite le mélange pendant une nuit à température ambiante, on évapore le solvant, on reprend le résidu avec 200 ml d'éther et 100 ml d'eau, on sépare la phase organique, on la lave trois fois avec 100 ml d'eau puis avec 100 ml de solution saturée de chlorure de sodium, on la sèche sur sulfate de magnésium et on l'évapore. On purifie le résidu par chromatographie sur colonne de silice et on obtient 6,4 g de produit sirupeux.

On en dissout 6,3 g dans 145 ml d'alcool isopropylique chlorhydrique 0,1N, on évapore la solution sous vide et on recristallise le résidu dans un mélange éthanol/diisopropyléther.

On obtient 5,2 g de chlorhydrate sous forme de poudre blanche. Point de fusion : 146-148°C.

Exemple 3 (Composé N°37)

N-[[[(diméthoxy-3,4 phényl)-2 éthyl]méthylamino]-4 butyl] N-[(méthyl-2 propoxy)-2 phényl] (trifluorométhyl)-3 benzamide, oxalate.

a) N-[(méthyl-2 propoxy)-2 phényl] (triflourométhyl)-3 benzamide.

A une suspension de 9,9 g de (méthyl-2 propoxy)-2 benzènamine et de 33 g de carbonate de potassium dans 90 ml de dichlorométhane, refroidie par un bain de glace, on ajoute goutte à goutte une solution de 12,5 g de chlorure de (trifluorométhyl)-3 benzoyle dans 60 ml de dichlorométhane.

On agite le mélange pendant une nuit à température ambiante, puis on évapore le solvant. On reprend le résidu avec un mélange de 200 ml d'eau et 300 ml d'éther, on sépare la phase organique, on la lave successivement avec deux fois 50 ml d'eau, deux fois 50 ml d'acide chlorhydrique aqueux 1N, trois fois 50 ml d'eau, et 25 ml de solution saturée de chlorure de sodium. On la sèche sur sulfate de magnésium, et on l'évapore sous vide. On obtient 19,7 g de produit sirupeux qu'on utilise tel quel dans l'étape suivante.

b) N-(chloro-4 butyl) N-[(méthyl-2 propoxy)-2 phényl](trifluorométhyle)-3 benzamide.

A une suspension de 4 g d'hydrure de lithium (à 50 % dans l'huile) dans 80 ml de diméthylformamide, refroidie par un bain de glace, on ajoute goutte à goutte les 19,7 g du produit précédent dissous dans 30 ml de diméthylformamide, puis 7,95 ml de bromo-1 chloro-4 butane.

On agite le mélange à température ambiante pendant une nuit, puis on ajoute 250 ml d'eau et 250 ml d'éther, on sépare la phase organique, on extrait la phase aqueuse une seconde fois avec 250 ml d'éther, on réunit les phases organiques en une seule, on lave cette dernière avec 100 ml d'eau, puis deux fois avec 100 ml d'acide chlorhydrique aqueux 0,5N, puis trois fois avec 50 ml d'eau, et enfin avec 50 ml de solution saturée de chlorure de sodium. On la sèche sur sulfate de magnésium et on l'évapore sous vide. On obtient 24,2 g de produit sirupeux qu'on utilise tel quel dans l'étape suivante.

c) N-[[[(diméthoxy-3,4 phényl)-2 éthyl]méthylamino]-4 butyl] N-[(méthyl-2 propoxy)-2 phényl] (trifluorométhyl)-3 benzamide.

On agite à 80°C, pendant 12 h, un mélange de 2,14 g du produit précédent, 1,46 g de N-[(diméthoxy-3,4 phényl)-2 éthyl]méthylamine, 2,07 g de carbonate de potassium, 0,8 g d'iodure de potassium, 0,09 g d'iodure de tétra-n-butylammonium dans 25 ml de diméthylformamide.

Puis on ajoute 250 ml d'éther et 50 ml de soude 0,5N, on sépare la phase organique, on la lave trois fois avec 50 ml d'eau puis avec 25 ml de solution saturée de chlorure de sodium, on la sèche sur sulfate de magnésium et on l'évapore. On purifie le résidu par chromatographie sur colonne de silice et on obtient 2,4 g de produit sirupeux.

On en dissout 2,15 g dans 150 ml d'éther et on ajoute goutte à goutte une solution de 0,33 g d'acide oxalique

dans 50 ml d'éther.

On laisse reposer une nuit à température ambiante, on sépare le préciité par filtration, on le lave à l'éther et on le recristallise dans un mélange de 40 ml d'alcool isopropylique et 260 ml de diisopropyléther.

On obtient 1,6 g d'oxalate sous forme de poudre blanche. Point de fusion : 80-82°C.

Le tableau ci-après illustre les structures et propriétés physiques de quelques composés selon l'invention.

Tableau

| Composé | $R_1$ | X | Ar | $R_2$ | $R_3$ | Sel | F(°C) |
|---|---|---|---|---|---|---|---|
| 1 | $-CH_2CH(CH_3)_2$ | H | $C_6H_5$ | $CH_3$ | $CH_3$ | 08 | 110-113 |
| 2 | $-CH_2CH(CH_3)_2$ | H | $C_6H_4$-3-$CF_3$ | $CH_3$ | $CH_3$ | 10 | 146-148 |
| 3 | $-CH_2CH(CH_3)_2$ | 4-F | $C_6H_4$-3-$CF_3$ | $CH_3$ | $CH_3$ | 10 | 119-120 |
| 4 | $-CH_2CH(CH_3)_2$ | 5-Cl | $C_6H_4$-3-$CF_3$ | $CH_3$ | $CH_3$ | 46 | 124-130 |
| 5 | $-CH_2C(CH_3)_3$ | H | $C_6H_4$-3-$CF_3$ | $CH_3$ | $CH_3$ | 46 | 116-120 |
| 6 | $-CH_2$-c$C_3H_5$ | H | $C_6H_4$-3-$CF_3$ | $CH_3$ | $CH_3$ | 10 | 118-120 |
| 7 | $-CH_2$-c$C_5H_9$ | H | $C_6H_4$-3-$CF_3$ | $CH_3$ | $CH_3$ | 10 | 142-144 |
| 8 | $-CH(CH_3)_2$ | H | $C_6H_4$-3-$CF_3$ | $CH_3$ | $CH_3$ | 10 | 134-136 |
| 9 | -c$C_5H_9$ | H | $C_6H_4$-3-$CF_3$ | $CH_3$ | $CH_3$ | 10 | 110-112 |
| 10 | $-CH_2CH(CH_3)_2$ | H | $C_6H_4$-2-F | $CH_3$ | $CH_3$ | 46 | 111-114 |
| 11 | $-CH_2CH(CH_3)_2$ | H | $C_6H_4$-2-Cl | $CH_3$ | $CH_3$ | 08 | 144-146 |
| 12 | $-CH_2CH(CH_3)_2$ | H | $C_6H_4$-2-$OCH_3$ | $CH_3$ | $CH_3$ | 08 | 150-152 |
| 13 | $-CH_2CH(CH_3)_2$ | H | $C_6H_4$-3-$NO_2$ | $CH_3$ | $CH_3$ | 46 | 146-148 |
| 14 | $-CH_2CH(CH_3)_2$ | H | $C_6H_4$-2-$NO_2$ | $CH_3$ | $CH_3$ | 46 | 112-114 |
| 15 | $-CH_2CH(CH_3)_2$ | H | $C_6H_4$-3-F | $CH_3$ | $CH_3$ | 10 | 122 |
| 16 | $-CH_2CH(CH_3)_2$ | H | $C_6H_4$-3-Cl | $CH_3$ | $CH_3$ | 10 | 135-136 |
| 17 | $-CH_2CH(CH_3)_2$ | H | $C_6H_4$-2-$CF_3$ | $CH_3$ | $CH_3$ | 46 | 133-134 |
| 18 | $-CH_2CH(CH_3)_2$ | H | $C_6H_4$-3-CN | $CH_3$ | $CH_3$ | 10 | 144-146 |
| 19 | $-CH_2CH(CH_3)_2$ | H | $C_6H_3$-2,3-$(CH_3)_2$ | $CH_3$ | $CH_3$ | 46 | 138-140 |
| 20 | $-CH_2CH(CH_3)_2$ | H | $C_6H_3$-2-$CH_3$-3-$NO_2$ | $CH_3$ | $CH_3$ | 46 | 148-150 |
| 21 | $-CH_2CH(CH_3)_2$ | H | $C_6H_3$-2-Cl-3-$NO_2$ | $CH_3$ | $CH_3$ | 08 | 114-116 |
| 22 | $-CH_2CH(CH_3)_2$ | H | $C_6H_3$-2,3-$Cl_2$ | $CH_3$ | $CH_3$ | 08 | 154-156 |
| 23 | $-CH_2CH(CH_3)_2$ | H | $C_6H_3$-2,5-$Cl_2$ | $CH_3$ | $CH_3$ | 08 | 130 |
| 24 | $-CH_2CH(CH_3)_2$ | H | $C_6H_3$-2-Cl-6-F | $CH_3$ | $CH_3$ | 08 | 152-154 |
| 25 | $-CH_2CH(CH_3)_2$ | H | $C_6H_3$-2-Cl-4-$NO_2$ | $CH_3$ | $CH_3$ | 10 | 190-192 |
| 26 | $-CH_2CH(CH_3)_2$ | H | $C_6H_3$-2-Cl-5-$NO_2$ | $CH_3$ | $CH_3$ | 10 | 166-168 |
| 27 | $-CH_2CH(CH_3)_2$ | H | $C_6H_3$-2,4-$Cl_2$ | $CH_3$ | $CH_3$ | 10 | 180-182 |
| 28 | $-CH_2CH(CH_3)_2$ | H | $C_6H_3$-2-$NO_2$-5-Cl | $CH_3$ | $CH_3$ | 10 | 140-142 |
| 29 | $-CH_2CH(CH_3)_2$ | H | $C_6H_3$-2-$OCH_3$-5-$NO_2$ | $CH_3$ | $CH_3$ | 10 | 126-130 |

Tableau (suite)

| Composé | $R_1$ | X | Ar | $R_2$ | $R_3$ | Sel | F(°C) |
|---|---|---|---|---|---|---|---|
| 30 | $-CH_2CH(CH_3)_2$ | H | $C_6H_3$-2-Cl-5-Br | $CH_3$ | $CH_3$ | 10 | 186-188 |
| 31 | $-CH_2CH(CH_3)_2$ | H | $C_6H_3$-3,5-$Cl_2$ | $CH_3$ | $CH_3$ | 46 | 156-158 |
| 32 | $-CH_2CH(CH_3)_2$ | H | $C_6H_3$-3-$NO_2$-4-Cl | $CH_3$ | $CH_3$ | 46 | 114-116 |
| 33 | $-CH_2CH(CH_3)_2$ | H | $C_6H_3$-3,4-$Cl_2$ | $CH_3$ | $CH_3$ | 46 | 139-141 |
| 34 | $-CH_2CH(CH_3)_2$ | H | $C_6H_5$ | $-(CH_2)_4-$ | | 46 | 110-112 |
| 35 | $-CH_2CH(CH_3)_2$ | H | $C_6H_4$-4-$OCH_3$ | $-(CH_2)_4-$ | | 46 | 149 |
| 36 | $-CH_2CH(CH_3)_2$ | H | $C_6H_4$-3-$CF_3$ | 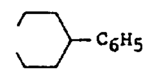 | | 46 | 134-136 |
| 37 | $-CH_2CH(CH_3)_2$ | H | $C_6H_4$-3-$CF_3$ | $CH_3$ | $CH_2CH_2$- 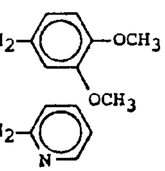 | 46 | 80-82 |
| 38 | $-CH_2CH(CH_3)_2$ | H | $C_6H_4$-3-$CF_3$ | $CH_3$ | $CH_2CH_2$- | 2·10 | 146-149 |
| 39 | $-CH_2CH(CH_3)_2$ | H | $C_6H_4$-3-$CF_3$ | $CH_3$ | $CH_2C_6H_5$ | 46 | 160-162 |
| 40 | $-CH_2CH(CH_3)_2$ | H | $C_6H_4$-3-$CF_3$ | 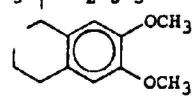 | | 15 | 142-144 |
| 41 | $-CH_2CH(CH_3)_2$ | H | naphtyle-1 | $CH_3$ | $CH_3$ | 46 | 178-180 |
| 42 | $-CH_2CH(CH_3)_2$ | H | isoquinoléinyle-1 | $CH_3$ | $CH_3$ | 46 | 120-122 |
| 43 | $-CH_2CH(CH_3)_2$ | H | quinoléinyle-4 | $CH_3$ | $CH_3$ | 46 | 146-148 |
| 44 | $-CH_2CH(CH_3)_2$ | 4-$CH_3$ | naphtyle-1 | $CH_3$ | $CH_3$ | ½08 | 150-151 |
| 45 | $-CH_2CH(CH_3)_2$ | 4-F | naphtyle-1 | $CH_3$ | $CH_3$ | ½08 | 174-176 |
| 46 | $-CH_2CH(CH_3)_2$ | 5-Cl | naphtyle-1 | $CH_3$ | $CH_3$ | 10 | 104-108 |
| 47 | $-CH_2CH(CH_3)_2$ | 4-$OCH_3$ | naphtyle-1 | $CH_3$ | $CH_3$ | ½08 | 152-154 |
| 48 | $-CH_2CH(CH_3)_2$ | 5-$CH_3$ | naphtyle-1 | $CH_3$ | $CH_3$ | 46 | 114-118 |
| 49 | $-CH_2CH(CH_3)_2$ | 5-$CF_3$ | naphtyle-1 | $CH_3$ | $CH_3$ | 46 | 156-158 |
| 50 | $-CH_2CH(CH_3)_2$ | H | naphtyle-1 | $C_3H_7$ | $C_3H_7$ | 10 | 120 |
| 51 | $-CH_2CH(CH_3)_2$ | H | naphtyle-1 | $-(CH_2)_5-$ | | 10 | 198-200 |
| 51a | $-CH_2CH(CH_3)_2$ | H | naphtyle-2 | $CH_3$ | $CH_3$ | 08 | 152-154 |
| 51b | $-CH_2CH(CH_3)_2$ | H | pyridinyle-3 | $CH_3$ | $CH_3$ | 10 | 104-106 |

Tableau (suite)

| Composé | $R_1$ | X | Ar | $R_2$ | $R_3$ | Sel | F(°C) |
|---|---|---|---|---|---|---|---|
| 52 | $-CH_2CH(CH_3)_2$ | H | naphtyle-1 | | 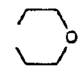 | 10 | 188-190 |
| 53 | $-CH_2CH(CH_3)_2$ | H | naphtyle-1 | | 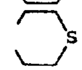 | 10 | 214-216 |
| 54 | $-CH_2CH(CH_3)_2$ | H | naphtyle-1 | | 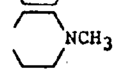 | 10 | 110-120 |
| 55 | $-CH_2CH(CH_3)_2$ | H | naphtyle-1 | $CH_3$ | 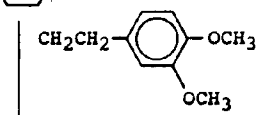 | 46 | 87-93 |
| 56 | $-CH_2CH(CH_3)_2$ | H | $C_6H_3$-2-Cl-5-$NO_2$ | $CH_3$ | 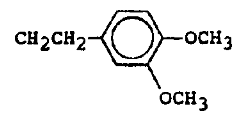 | 46 | 143-145 |
| 57 | $-CH_2CH(CH_3)_2$ | H | $C_6H_4$-3-$CF_3$ | $CH_3$ | 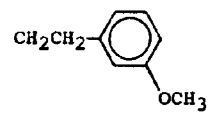 | 46 | 136-138 |
| 58 | $-CH_2CH(CH_3)_2$ | H | $C_6H_4$-3-$CF_3$ | $CH_3$ | 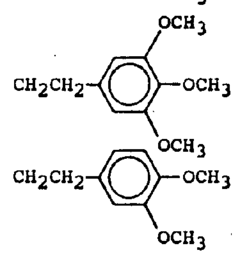 | 10 | 151-153 |
| 59 | $-CH_2CH(CH_3)_2$ | H | $C_6H_4$-4-$CF_3$ | $CH_3$ | | 46 | 128-130 |
| 60 | $-CH_2CH(CH_3)_2$ | H | $C_6H_3$-2-$OCH_3$-5-$NO_2$ | $CH_3$ | 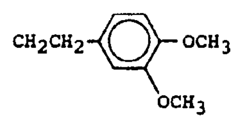 | 10 | 158-160 |

Tableau (suite)

| Composé | $R_1$ | X | Ar | $R_2$ | $R_3$ | Sel | F(°C) |
|---|---|---|---|---|---|---|---|
| 61 | $-CH_2CH(CH_3)_2$ | H | $C_6H_3$-2,5-$Cl_2$ | $CH_3$ | 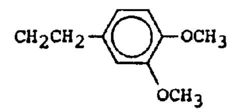 | 10 | 78-80 |
| 62 | $-CH_2CH(CH_3)_2$ | H | $C_6H_3$-2-$CH_3$-3-$NO_2$ | $CH_3$ | 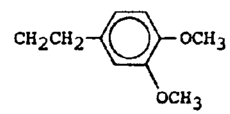 | 10 | 104-106 |
| 63 | $-CH_2CH(CH_3)_2$ | H | $C_6H_3$-3,4-$(OCH_3)_2$ | $CH_3$ | 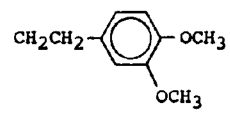 | 46 | 81-83 |
| 64 | $-CH_2CH(CH_3)_2$ | H | $C_6H_3$-3,4-$OCH_2O$- | $CH_3$ | 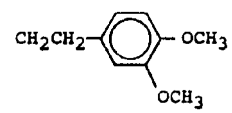 | 46 | 133-134 |
| 65 | $-CH_2CH(CH_3)_2$ | H | $C_6H_2$-3,4,5-$(OCH_3)_3$ | $CH_3$ | 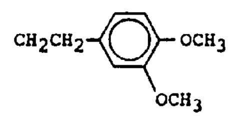 | 46 | 90-94 |
| 66 | $-CH_2CH(CH_3)_2$ | H | $C_6H_4$-3-$NO_2$ | $CH_3$ | 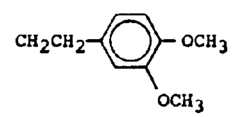 | 10 | 120-122 |
| 67 | $-CH_2CH(CH_3)_2$ | H | $C_6H_3$-2,3-$(CH_3)_2$ | $CH_3$ | 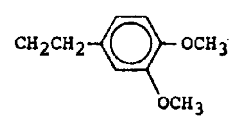 | 46 | 80-84 |

Tableau (suite)

| Composé | $R_1$ | X | Ar | $R_2$ | $R_3$ | Sel | F(°C) |
|---|---|---|---|---|---|---|---|
| 68 | $-CH_2CH(CH_3)_2$ | H | $C_6H_3$-2,3-$Cl_2$ | $CH_3$ | 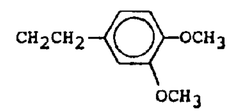 | 46 | 98-100 |
| 69 | $-CH(CH_3)_2$ | H | $C_6H_4$-3-$CF_3$ | $CH_3$ | 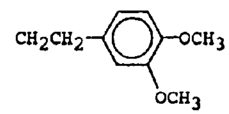 | 46 | 115-117 |
| 70 | $-C(CH_3)_3$ | H | $C_6H_4$-3-$CF_3$ | $CH_3$ | 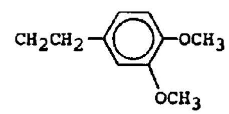 | 46 | 128-130 |
| 71 | $-cC_5H_9$ | H | $C_6H_4$-3-$CF_3$ | $CH_3$ | 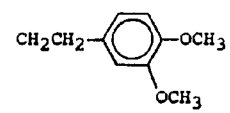 | 46 | 132-134 |
| 72 | $-CH_2CH(CH_3)_2$ | H | $C_6H_4$-3-$CF_3$ | H | 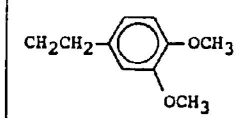 | 08 | 132-134 |
| 73 | $-CH_2CH(CH_3)_2$ | H | $C_6H_4$-3-$CF_3$ | $CH_3$ | 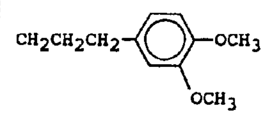 | 46 | 66-70 |
| 74 | $-CH_2CH(CH_3)_2$ | H | $C_6H_4$-3-$CF_3$ | $CH_3$ | 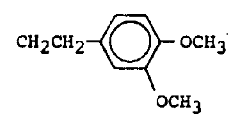 | 46 | 122-124 |

Tableau (fin)

| Composé | $R_1$ | X | Ar | $R_2$ | $R_3$ | Sel | F(°C) |
|---|---|---|---|---|---|---|---|
| 75 | $-CH_2CH(CH_3)_2$ | H | $C_6H_4$-3-$CF_3$ | $CH_3$ | $CH_2CH_2$- 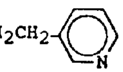 | 2·46<br>2·10 | 112-116<br>140-150 |
| 76 | $-CH_2CH(CH_3)_2$ | H | $C_6H_4$-3-$CF_3$ | $CH_3$ | $CH_2CH_2$- 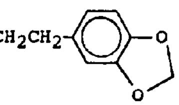 | 10 | 138-140 |
| 77 | $-CH_2CH(CH_3)_2$ | H | $C_6H_4$-3-$CF_3$ | $CH_3$ | $CH_2CH_2$- 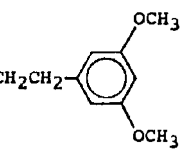 | 46 | 122-124 |
| 78 | $-CH_2CH(CH_3)_2$ | H | $C_6H_4$-3-$CF_3$ | $CH_3$ | $CH_2CH_2$- 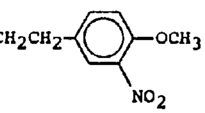 | 46 | 104-108 |
| 79 | $-CH_2CH(CH_3)_2$ | H | $C_6H_4$-3-$CF_3$ | $CH_3$ | $CH_2CH_2$- 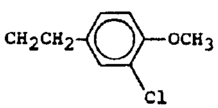 | 10 | 120-124 |
| 80 | $-CH_2CH(CH_3)_2$ | H | $C_6H_4$-3-$CF_3$ | $CH_3$ | $CH_2CH_2$- 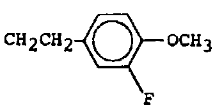 | 10 | 114-118 |
| 81 | $-CH_2CH(CH_3)_2$ | H | $C_6H_4$-3-$CF_3$ | $CH_3$ | $CH_2CH_2$- 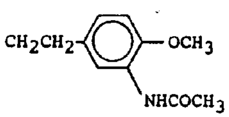 | 46 | 88-90 |

Légende du tableau

Colonne "$R_1$" : $cC_3H_5$ et $cC_5H_9$ désignent les groupes cyclopropyle et cyclopentyle, respectivement.

Colonne "Ar" : $C_6H_5$, $C_6H_4$, $C_6H_3$ et $C_6H_2$ désignent des groupes phényle non substitué, monosubstitué, disubstitué et trisubstitué, respectivement. Les formules des substituants sont indiquées, précédées du numéro de leur(s) position(s).

Colonne "Sel" :

08 désigne le fumarate
$\frac{1}{2}$08 désigne l'hémifumarate
10 désigne le chlorhydrate
2·10 désigne le dichlorhydrate
15 désigne le méthanesulfonate
46 désigne l'oxalate
2·46 désigne le dioxalate

Les composés de l'invention ont fait l'objet de divers essais pharmacologiques qui ont mis en évidence leur intérêt comme susbstances à activités thérapeutiques.

Ainsi par exemple leur action antagoniste des effets du calcium a été étudiée sur l'aorte isolée du lapin. Le protocole expérimental utilisé est une variante de celui de Godfraind et Kaba (Blockade or reversal of the contraction induced by calcium and adrenaline in depolarized arterial smooth muscle, Br. J. Pharmac., (1969), 36, 549-560). Les expériences sont réalisées sur des tronçons d'aorte thoracique de lapin. Les animaux, des "Fauves de Bourgogne" d'un poids moyen de 1,5 kg, sont sacrifiés par dislocation cervicale et exsanguination. L'aorte thoracique est rapidement prélevée et placée dans un milieu de Krebs bicarbonaté et oxygéné (95% $0_2$ + 5% $CO_2$).

Des tronçons d'aorte de 1 cm de long environ sont préparés et installés dans des cuves à organes de 20 ml contenant de la solution de Krebs bicarbonatée et oxygénée à pH 7,4 à 37°C. Deux crochets métalliques en "U" de la longueur des tronçons sont introduits dans la lumière de ceux-ci. L'un des crochets est fixé à la base de la cuve et l'autre, relié à une jauge de contrainte isométrique (Grass FT03), permet l'enregistrement, par l'intermédiaire d'un préamplificateur continu (Grass 7P1), des réponses contractiles des tronçons d'aorte sur un oscillographe à encre (Grass 79B). Cette méthode présente, par rapport aux préparations en spirale ou en anneaux, l'avantage de mieux respecter l'intégrité structurale des vaisseaux et de n'enregistrer que la composante radiale des réponses contractiles, qui représente le phénomène intéressant du point de vue fonctionnel (régulation de la pression artérielle). Une tension initiale de 4 g est imposée aux préparations.

De la phénoxybenzamine (1 μM) et du propranolol (1 μM) sont ajoutés aux différents milieux de Krebs afin de supprimer les réponses contractiles liées à l'activation des récepteurs α-et β-adrénergiques vasculaires.

Après une heure de stabilisation dans le milieu de Krebs, la tension imposée aux aortes est ramenée à 2 g puis, après une période d'attente de 30 minutes, les préparations sont incubées pendant une dizaine de minutes dans une solution de Krebs bicarbonatée sans calcium en présence d'EDTA (200 μM) et de propranolol (1 μM). Cette solution est alors remplacée par un milieu de Krebs dépolarisant (riche en potassium) sans calcium et contenant du propranolol (1 μM). Après 5 minutes, une concentration unique de 1 mM de calcium est ajoutée à cette solution et une période de stabilisation de 30 minutes est respectée, qui permet aux préparations d'atteindre une contraction stable.

Ensuite des doses cumulatives des composés à étudier sont administrées toutes les 30 minutes (temps généralement nécessaire pour l'obtention d'un plateau) jusqu'à disparition totale de la contraction provoquée par 1 mM de calcium ou bien jusqu'à la concentration (30 μM) de produit à étudier. En fin d'expérience une concentration supramaximale de papavérine (300 μM) est administrée afin de déterminer la décontraction maximale possible de chaque préparation.

Les valeurs absolues (en grammes) de la contraction initiale (après 1 mM de chlorure de calcium) et de la contraction après les différentes concentrations cumulatives de composés vasodilatateurs sont obtenues, pour chaque préparation, par différence avec la contraction minimale observée 30 minutes après l'addition finale de 300 μM de papavérine. Le pourcentage de diminution de la contraction, par rapport à la contraction provoquée par 1 mM de calcium, est calculé pour chaque dose de composé et chaque préparation, et la moyenne $\overline{X}$ ± E.S.M. des pourcentages individuels est calculée. Les moyennes obtenues (pondérées par l'inverse de l'Erreur Standard à la Moyenne), sont analysées à l'aide d'un modèle mathématique de courbe sigmoïde, et la concentration molaire provoquant 50% de décontraction de la réponse au calcium ($CE_{50}$) est calculée.

Pour les composés de l'invention les $CE_{50}$ vont de 0,2 à 10 μM.

Les composés de l'invention ont également fait l'objet d'un essai de liaison (binding) de [$^3$H]nitrendipine sur le cortex total de rat.

On utilise des rats mâles Sprague-Dawley de 150 à 200 g. Après dislocation cervicale on excise le cerveau

et on dissèque le cortex cérébral sur une boîte de culture refroidie par de la glace. On le place dans 20 volumes d'une solution tampon à 50 mM de tris(hydroxyméthyle)aminométhane, glacée, dont le pH a été ajusté à 7,4 au moyen d'acide chlorhydrique (tampon "Tris-Hcl"). On homogénéise le tissu à l'aide d'un appareil Polytron Ultra-Turrax pendant 30 secondes à la moitié de la vitesse maximale, et on lave les préparations trois fois avec la solution tampon glacée, en les essorant chaque fois par centrifugation à 49000xg pendant 10 minutes. Finalement on prépare des suspensions à 100 mg de tissu pour 1 ml de tampon Tris-Hcl à 50 mM (pH=7,4 à 37°C). On fait ensuite incuber des portions aliquotes de 100 μl de suspension de membranes lavées avec de la [$^3$H]nitrendipine (New England Nuclear, d'une activité spécifique de 70,0 Ci/mmole) dans un volume final de 1 ml de tampon Tris-HCl. Après 30 minutes d'incubation à 37°C on récupère les membranes par filtration sur fibres de verre Whatman GF/F, on les lave trois fois avec 5 ml de tampon Tris-Hcl glacé. On mesure la quantité de radioactivité liée au tissu et retenue sur les filtres par spectrométrie de scintillation. La liaison spécifique de la [$^3$H]nitrendipine est définie comme la diminution de la quantité de radioligand retenue sur le filtre, due à l'introduction de 1 μM de nifedipine pendant l'incubation. La liaison spécifique représente 80 à 90% de la quantité totale de la radioactivité recueillie sur le filtre. A l'aide de différentes concentrations de composés à étudier, on détermine graphiquement la concentration $CI_{50}$, concentration du composé étudié qui inhibe 50 % de la liaison spécifique de la [$^3$H]nitrendipine.
Les concentrations $CI_{50}$ des composés de l'invention se situent entre 0,005 et 0,1 μM.

Enfin les composés de l'invention ont été étudiés quant à leur effet antihypertenseur chez le rat spontanément hypertendu.
On mesure la pression systolique grâce à un cathéter placé dans l'artère caudale, selon la méthode de Gerold et Tschirky (Arzneim.-Forsch., 1968, 18, 1285-1287), et on enregistre les variations de pression en fonction du temps écoulé, pour chaque composé étudié et pour chaque dose administrée.
Les composés de l'invention provoquent une diminution de la pression sanguine de 15 à 30% au bout de 30 minutes et de 20 à 25% au bout de 3 heures, à des doses de 5 à 30 mg/kg administrées par la voie intrapéritonéale.

Les résultats des essais pharmacologiques montrent que les composés de l'invention sont des antagonistes du calcium et peuvent, à ce titre, être utilisés pour le traitement de diverses affections pour lesquelles ce type d'agents est indiqué.
C'est ainsi qu'en particulier ils peuvent être utilisés en médecine cardiovasculaire pour le traitement d'affections nécessitant des modulateurs des mouvements transmembranaires et intracellulaires du calcium, tout spécialement l'hypertension, l'angor et l'arythmie cardiaque.
Ils sont en outre susceptibles de présenter des effets antiathérogénes, antiagrégants plaquettaires, anti-ischémiques cardiaques, anti-ischémiques cérébraux, antimigraineux, antiépileptiques, antiasthmatiques et antiulcéreux.
Dans le domaine cardiovasculaire ils peuvent être utilisés seuls ou associés à d'autres substances actives connues telles que les diurétiques, les β-bloquants, les inhibiteurs de l'enzyme de conversion de l'angiotensine, les antagonistes des récepteurs $\alpha_1$.
Combinés avec des agents destinés à potentialiser leurs effets ou à diminuer leur toxicité, ils peuvent être également indiqués pour le traitement du cancer ou dans les transplantations.

Les composés de l'invention peuvent être présentés sous toutes formes appropriées à l'administration orale ou parentérale, en association avec des excipients connus, par exemple sous forme de comprimés, gélules, dragées, capsules, solutions ou suspensions buvables ou injectables.
La posologie journalière peut aller par exemple de 5 à 200 mg par la voie orale et de 0,1 à 10 mg par voie parentérale.

## Revendications

### Revendications pour les Etats contractants suivants : AT, BE, CH, DE, FR, GB, IT, LI, LU, NL, SE.

1. Composés de formule générale (I)

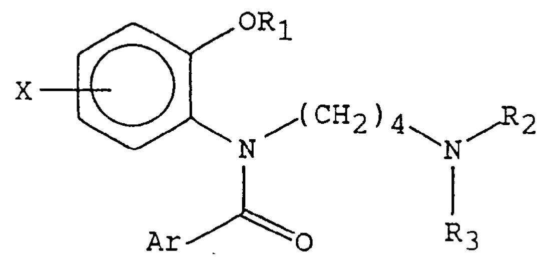

(I)

dans laquelle
X représente un atome d'hydrogène ou d'halogène ou un groupe trifluorométhyle, alkyle en $C_1$-$C_4$ ou alcoxy en $C_1$-$C_4$,
$R_1$ représente un groupe alkyle droit ou ramifié en $C_2$-$C_8$, un groupe cycloalkyle en $C_3$-$C_5$ ou un groupe cycloalkylméthyle dont le cycle est en $C_3$-$C_5$,
$R_2$, pris isolément, représente un atome d'hydrogène ou un groupe alkyle en $C_1$-$C_4$,
$R_3$, pris isolément, représente un atome d'hydrogène ou un groupe alkyle en $C_1$-$C_4$, un groupe phénylalkyle éventuellement substitué ou un groupe pyridinylalkyle,
ou bien,
$R_2$ et $R_3$, pris ensemble, représentent avec l'atome d'azote qui les porte un cycle pyrrolidinyle, pipéridinyle, morpholinyle, perhydrothiazinyle, pipérazinyle ou méthyl-4 pipérazinyle, et
Ar représente

- soit un groupe phényle portant éventuellement de un à trois substituants choisis parmi les atomes d'halogène, les groupes alkyle en $C_1$-$C_4$, alcoxy en $C_1$-$C_4$, trifluorométhyle, nitro et cyano,
- soit un groupe naphtyle, pyridinyle, quinoléinyle ou isoquinoléinyle,

ainsi que leurs sels d'addition à des acides acceptables en pharmacologie.

2. Composés selon la revendication 1, caractérisés en ce que $R_1$ représente un groupe isobutyle et Ar représente un groupe phényle substitué.

3. Composés selon la revendication 2, caractérisés en ce que X représente un atome d'hydrogène et $R_2$ représente un groupe méthyle.

4. Composé selon la revendication 3, caractérisé en ce que $R_3$ représente un groupe méthyle et Ar représente un groupe diméthyl-2,3 phényle.

5. Composé selon la revendication 3, caractérisé en ce que $R_3$ représente un groupe méthyle et Ar représente un groupe trifluorométhyl-3 phényle.

6. Composé selon la revendication 3, caractérisé en ce que $R_3$ représente un groupe méthyle et Ar représente un groupe nitro-3 phényle.

7. Composé selon la revendication 3, caractérisé en ce que $R_3$ représente un groupe méthyle et Ar représente un groupe méthoxy-2 nitro-5 phényle.

8. Composé selon la revendication 3, caractérisé en ce que $R_3$ représente un groupe (méthoxy-3 phényle)-2 éthyle et Ar représente un groupe trifluorométhyl-3 phényle.

9. Composé selon la revendication 3, caractérisé en ce que $R_3$ représente un groupe (diméthoxy-3,4 phényl)-2 éthyle et Ar représente un groupe trifluorométhyl-3 phényle.

10. Composé selon la revendication 3, caractérisé en ce que $R_3$ représente un groupe (pyridinyl-3)-2 éthyle et Ar représente un groupe trifluorométhyl-3 phényle.

11. Composé selon la revendication 3, caractérisé en ce que $R_3$ représente un groupe (diméthoxy-3,4 phényl)-2 éthyle et Ar représente un groupe méthyl-2 nitro-3 phényle.

12. Procédé de préparation des composés selon la revendication 1, caractérisé en ce qu'on soumet une diamine de formule (II)

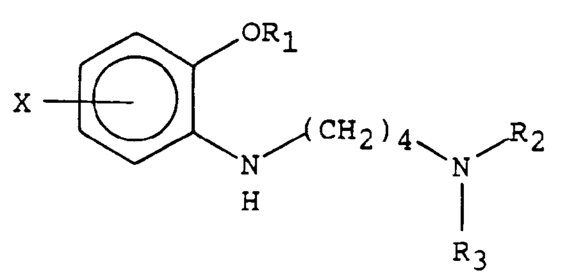

(II)

dans laquelle X, $R_1$, $R_2$ et $R_3$ sont tels que définis dans la revendication 1, à une acylation au moyen d'un chlorure d'acide de formule (III)

ArCOCl (III)

dans laquelle Ar est tel que défini dans la revendication 1.

13. Procédé de préparation des composés selon la revendication 1, caractérisé en ce qu'on soumet une benzènamine de formule (IV)

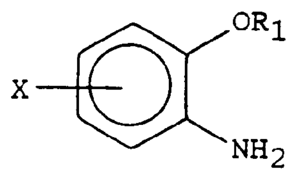

(IV)

dans laquelle X et $R_1$ sont tels que définis dans la revendication 1, à une acylation au moyen d'un chlorure d'acide de formule (III)

ArCOCl (III)

dans laquelle Ar est telle que défini dans la revendication 1, pour obtenir un amide de formule (XIII)

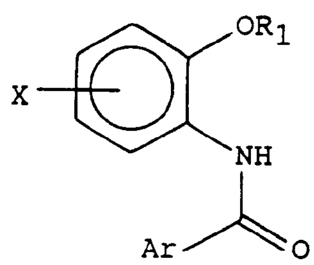

(XIII)

que l'on fait réagir avec le bromo-1 chloro-4 butane, puis on traite le composé de formule (XIV) ainsi obtenu

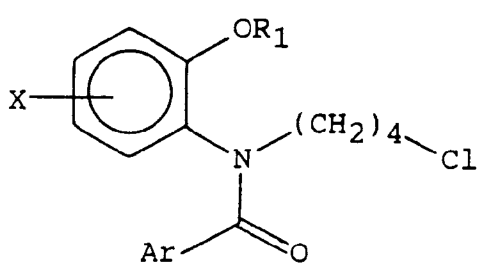

(XIV)

avec une amine de formule (VII)

$HNR_2R_3$ (VII)

dans laquelle $R_2$ et $R_3$ sont tels que définis dans la revendication 1.

14. Composition pharmaceutique, caractérisée en ce qu'elle contient un composé selon l'une des revendications 1 à 11, associé à un excipient pharmaceutique.

**Revendications pour les Etats contractants suivants: ES, GR**

1. Procédé de préparation des composés de formule générale (I)

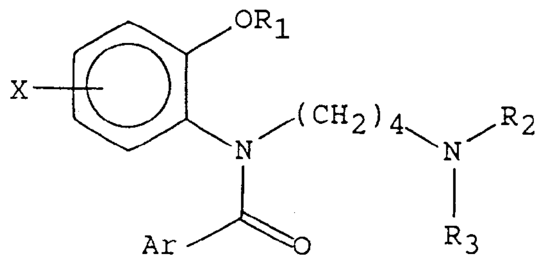

(I)

dans laquelle
X représente un atome d'hydrogène ou d'halogène ou un groupe trifluorométhyle, alkyle en $C_1$-$C_4$ ou alcoxy en $C_1$-$C_4$,
$R_1$ représente un groupe alkyle droit ou ramifié en $C_2$-$C_8$, un groupe cycloalkyle en $C_3$-$C_5$ ou un groupe cycloalkylméthyle dont le cycle est en $C_3$-$C_5$,
$R_2$, pris isolément, représente un atome d'hydrogène ou un groupe alkyle en $C_1$-$C_4$,
$R_3$, pris isolément, représente un atome d'hydrogène ou un groupe alkyle en $C_1$-$C_4$, un groupe phénylalkyle éventuellement substitué ou un groupe pyridinylalkyle,
ou bien,
$R_2$ et $R_3$, pris ensemble, représentent avec l'atome d'azote qui les porte un cycle pyrrolidinyle, pipéridinyle, morpholinyle, perhydrothiazinyle, pipérazinyle ou méthyl-4 pipérazinyle, et
Ar représente

- soit un groupe phényle portant éventuellement de un à trois substituants choisis parmi les atomes d'halogène, les groupes alkyle en $C_1$-$C_4$, alcoxy en $C_1$-$C_4$, trifluorométhyle, nitro et cyano,
- soit un groupe naphtyle, pyridinyle, quinoléinyle ou isoquinoléinyle,

procédé caractérisé en ce qu'on soumet une diamine de formule (II)

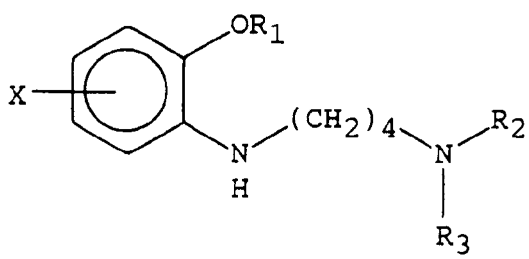

(II)

dans laquelle X, $R_1$, $R_2$ et $R_3$ sont tels que définis ci-dessus à une acylation au moyen d'un chlorure d'acide de formule (III)

ArCOCl (III)

dans laquelle Ar est tel que défini ci-dessus.
2. Procédé de préparation des composés de formule générale

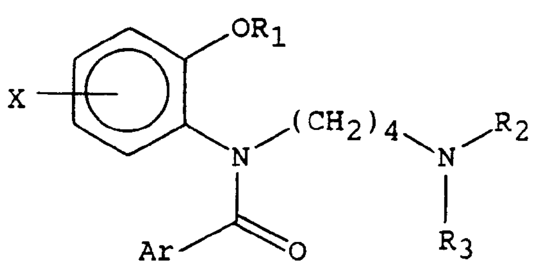

(I)

(I) danns laquelle
X représente un atome d'hydrogène ou d'halogène ou un groupe trifluorométhyle, alkyle en $C_1$-$C_4$ ou alcoxy en $C_1$-$C_4$,
$R_1$ représente un groupe alkyle droit ou ramifié en $C_2$-$C_8$, un groupe cycloalkyle en $C_3$-$C_5$ ou un groupe cycloalkylméthyle dont le cycle est en $C_3$-$C_5$,

$R_2$, pris isolément, représente un atome d'hydrogène ou un groupe alkyle en $C_1$-$C_4$,
$R_3$, pris isolément, représente un atome d'hydrogène ou un groupe alkyle en $C_1$-$C_4$, un groupe phénylalkyle éventuellement substitué ou un groupe pyridinylalkyle,
ou bien,
$R_2$ et $R_3$, pris ensemble, représentent avec l'atome d'azote qui les porte un cycle pyrrolidinyle, pipéridinyle, morpholinyle, perhydrothiazinyle, pipérazinyle ou méthyl-4 pipérazinyle, et
Ar représente

- soit un groupe phényle portant éventuellement de un à trois substituants choisis parmi les atomes d'halogène, les groupes alkyle en $C_1$-$C_4$, alcoxy en $C_1$-$C_4$, trifluorométhyle, nitro et cyano,
- soit un groupe naphtyle, pyridinyle, quinoléinyle ou isoquinoléinyle,

procédé caractérisé en ce qu'on soumet une benzènamine de formule (IV)

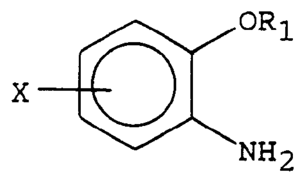

(IV)

dans laquelle X et $R_1$ sont tels que définis ci-dessus, à une acylation au moyen d'un chlorure d'acide de formule (III)

ArCOCl (III)

dans laquelle Ar est telle que défini ci-dessus, pour obtenir un amide de formule (XIII)

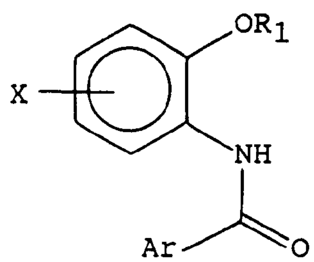

(XIII)

que l'on fait réagir avec le bromo-1 chloro-4 butane, puis on traite le composé de formule (XIV) ainsi obtenu

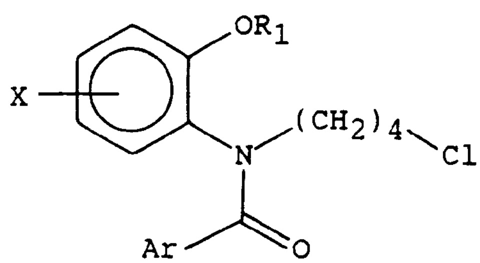

(XIV)

avec une amine de formule (VII)

$HNR_2R_3$ (VII)

dans laquelle $R_2$ et $R_3$ sont tels que définis ci-dessus.

## Patentansprüche

### Patentansprüche für folgende Vertragsstaaten : AT, BE, CH, DE, FR, GB, IT, LI, LU, NL, SE.

1. Verbindungen der allgemeinen Formel (I)

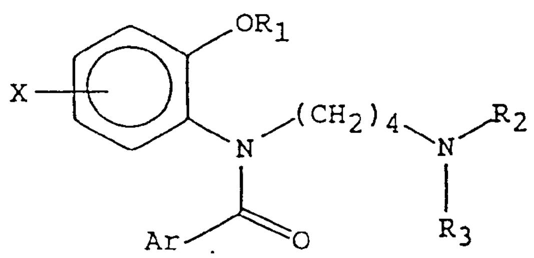

(I)

in welcher
X ein Wasserstoff- oder Halogenatom oder eine Trifluormethyl-, ($C_1$-$C_4$)-Alkyl- oder ($C_1$-$C_4$)-Alkoxygruppe,
$R_1$ eine geradkettige oder verzweigte ($C_2$-$C_8$)-Alkyl-, eine ($C_3$-$C_5$)-Cycloalkyl- oder eine Cycloalkylmethylgruppe mit einem ($C_3$-$C_5$)-Ring,
$R_2$ allein für sich ein Wasserstoffatom oder eine ($C_1$-$C_4$)-Alkylgruppe,
$R_3$ allein für sich ein Wasserstoffatom oder eine ($C_1$-$C_4$)-Alkylgruppe, eine gegebenenfalls substituierte Phenylalkylgruppe oder eine Pyridinylalkylgruppe bedeutet oder aber
$R_2$ und $R_3$ gemeinsam mit dem Stickstoffatom, an das sie gebunden sind, einen Pyrrolidinyl-, Piperidinyl-, Morpholinyl-, Perhydrothiazinyl-, Piperazinyl- oder 4-Methylpiperazinylring darstellen und
Ar entweder

- für eine Phenylgruppe mit gegebenenfalls ein bis drei Substituentpn aus der Gruppe der Halogenatome, der ($C_1$-$C_4$)-Alkylgruppen, ($C_1$-$C_4$)-Alkoxygruppen, der Methylendioxy-, Trifluormethyl-, Nitro- und Cyanogruppe,
- oder für eine Naphthyl-, Pyridinyl-, Chinolinyl- oder Isochinolinylgruppe steht,

sowie deren Additionssalze mit pharmakologisch verwendbaren Säuren.

2. Verbindungen nach Anspruch 1, dadurch gekennzeichnet, daß $R_1$ für eine Isobutyl- und Ar für eine substituierte Phenylgruppe steht.

3. Verbindungen nach Anspruch 2, dadurch gekennzeichnet, daß X für ein Wasserstoffatom und $R_2$ für eine Methylgruppe steht.

4. Verbindung nach Anspruch 3, dadurch gekennzeichnet, daß $R_3$ für eine Methylgruppe und Ar für eine 2,3-Dimethylphenylgruppe steht.

5. Verbindung nach Anspruch 3, dadurch gekennzeichnet, daß $R_3$ für eine Methylgruppe und Ar für eine 3-Trifluormethylphenylgruppe steht.

6. Verbindung nach Anspruch 3, dadurch gekennzeichnet, daß $R_3$ für eine Methylgruppe und Ar für eine 3-Nitrophenylgruppe steht.

7. Verbindung nach Anspruch 3, dadurch gekennzeichnet, daß $R_3$ für eine Methylgruppe und Ar für eine 2-Methoxy-5-nitrophenylgruppe steht.

8. Verbindung nach Anspruch 3, dadurch gekennzeichnet, daß $R_3$ für eine 2-(3-Methoxyphenyl)-ethylgruppe und Ar für eine 3-Trifluormethylphenylgruppe steht.

9. Verbindung nach Anspruch 3, dadurch gekennzeichnet, daß $R_3$ für eine 2-(3,4-Dimethoxyphenyl)-ethylgruppe und Ar für eine 3-Trifluormethylphenylgruppe steht.

10. Verbindung nach Anspruch 3, dadurch gekennzeichnet, daß $R_3$ für eine 2-(Pyridin-3-yl)-ethylgruppe und Ar für eine 3-Trifluormethylphenylgruppe steht.

11. Verbindung nach Anspruch 3, dadurch gekennzeichnet, daß $R_3$ für eine 2-(3,4-Dimethoxyphenyl)-ethylgruppe und Ar für eine 2-Methyl-3-nitrophenylgruppe steht.

12. Verfahren zur Herstellung von Verbindungen nach Anspruch 1, dadurch gekennzeichnet, daß man ein Diamin der Formel (II)

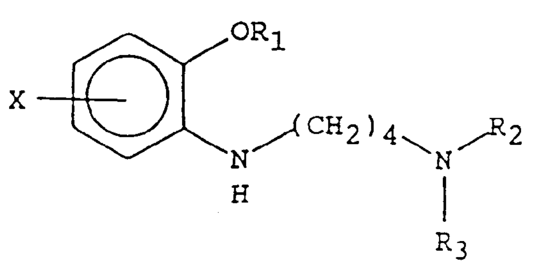

(II)

worin X, $R_1$, $R_2$ und $R_3$ die in Anspruch 1 angegebene Bedeutung haben, einer Acylierung mittels eines Säurechlorids der Formel (III)

ArCOCl (III),

in welcher Ar die in Anspruch 1 angegebene Bedeutung hat, unterwirft.

13. Verfahren zur Herstellung von Verbindungen nach Anspruch 1, dadurch gekennzeichnet, daß man ein Benzamin der Formel (IV)

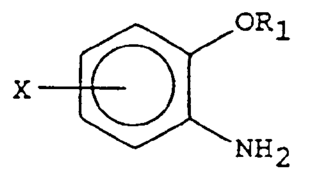

(IV)

in welcher X und $R_1$ die in Anspruch 1 genannte Bedeutung haben, einer Acylierung mittels eines Säurechlorids der Formel (III)

ArCOCl (III),

in welcher Ar die in Anspruch 1 angegebene Bedeutung hat, unterwirft, um ein Amid der Formel (XIII)

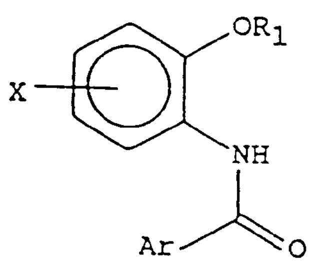

(XIII)

zu erhalten, das man mit 1-Brom-4-Chlorbutan umsetzt, worauf man die so erhaltene Verbindung der Formel (XIV)

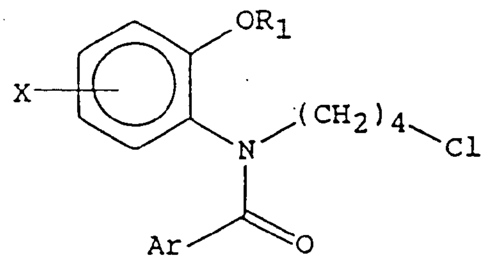

(XIV)

mit einem Amin der Formel (VII)

$HNR_2R_3$ (VII),

in welcher $R_2$ und $R_3$ die in Anspruch 1 angegebene Bedeutung haben, zur Reaktion bringt.

14. Pharmazeutische Zusammensetzung, dadurch gekennzeichnet, daß sie eine Verbindung nach einem der Ansprüche 1 bis 11 in Kombination mit einem geeigneten pharmazeutischen Trägermaterial enthält.

**Patentansprüche für folgende Vertragsstaaten : ES, GR**

1. Verfahren zur Herstellung von Verbindungen der allgemeinen Formel (I)

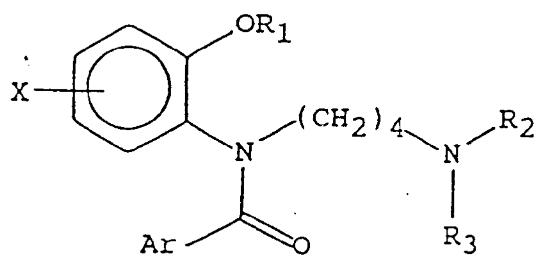

(I)

in welcher
X ein Wasserstoff- oder Halogenatom oder eine Trifluormethyl-, ($C_1$-$C_4$)-Alkyl- oder ($C_1$-$C_4$)-Alkoxygruppe,
$R_1$ eine geradkettige oder verzweigte ($C_2$-$C_8$)-Alkyl-, eine ($C_3$-$C_5$)-Cycloalkyl- oder eine Cycloalkylmethylgruppe mit einem ($C_3$-$C_5$)-Ring,
$R_2$ allein für sich ein Wasserstoffatom oder eine ($C_1$-$C_4$)-Alkylgruppe,
$R_3$ allein für sich ein Wasserstoffatom oder eine ($C_1$-$C_4$)-Alkylgruppe, eine gegebenenfalls substituierte Phenylalkylgruppe oder eine Pyridinylalkylgruppe bedeutet oder aber
$R_2$ und $R_3$ gemeinsam mit dem Stickstoffatom, an das sie gebunden sind, einen Pyrrolidinyl-, Piperidinyl-, Morpholinyl-, Perhydrothiazinyl-, Piperazinyl- oder 4-Methylpiperazinylring darstellen und
Ar entweder
- für eine Phenylgruppe mit gegebenenfalls ein bis drei Substituenten aus der Gruppe der Halogenatome, der ($C_1$-$C_4$)-Alkyl-gruppen, ($C_1$-$C_4$)-Alkoxygruppen, der Methylendioxy-, Trifluormethyl-, Nitro- und Cyanogruppe,
- oder für eine Naphthyl-, Pyridinyl-, Chinolinyl- oder Isochinolinylgruppe steht,

dadurch gekennzeichnet, daß man ein Diamin der Formel (II)

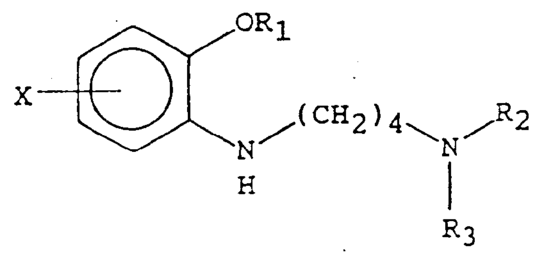

(II)

worin X, $R_1$, $R_2$ und $R_3$ die oben angegebene Bedeutung haben, einer Acylierung mittels eines Säurechlorids der Formel (III)

ArCOCl (III),

in welcher Ar die oben angegebene Bedeutung hat, unterwirft.

2. Verfahren zur Herstellung von Verbindungen der allgemeinen Formel (I)

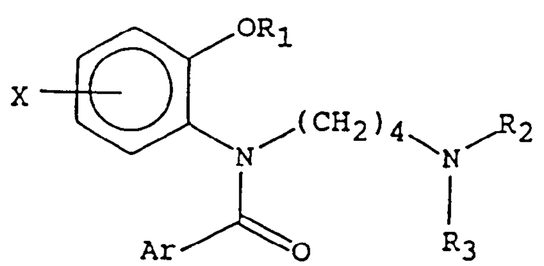

(I)

in welcher
X ein Wasserstoff- oder Halogenatom oder eine Trifluormethyl-, ($C_1$-$C_4$)-Alkyl- oder ($C_1$-$C_4$)-Alkoxygruppe,
$R_1$ eine geradkettige oder verzweigte ($C_2$-$C_8$)-Alkyl-, eine ($C_3$-$C_5$)-Cycloalkyl- oder eine Cycloalkylmethylgruppe mit einem ($C_3$-$C_5$)-Ring,
$R_2$ allein für sich ein Wasserstoffatom oder eine ($C_1$-$C_4$)-Alkylgruppe,
$R_3$ allein für sich ein Wasserstoffatom oder eine ($C_1$-$C_4$)-Alkylgruppe, eine gegebenenfalls substituierte Phenylalkylgruppe oder eine Pyridylalkylgruppe bedeutet oder aber

$R_2$ und $R_3$ gemeinsam mit dem Stickstoffatom, an das sie gebunden sind, einen Pyrrolidinyl-, Piperidinyl-, Morpholinyl-, Perhydrothiazinyl-, Piperazinyl- oder 4-Methylpiperazinylring darstellen und
Ar entweder

- für eine Phenylgruppe mit gegebenenfalls ein bis drei Substituenten aus der Gruppe der Halogenatome, der ($C_1$-$C_4$)-Alkylgruppen, ($C_1$-$C_4$)-Alkoxygruppen, der Methylendioxy-, Trifluormethyl-, Nitro- und Cyanogruppe,
- oder für eine Naphthyl-, Pyridinyl-, Chinolinyl- oder Isochinolinylgruppe steht,

dadurch gekennzeichnet, daß man ein Benzamin der Formel (IV)

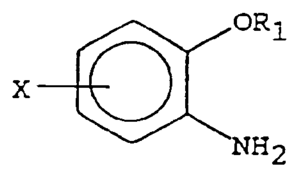

(IV)

in welcher X und $R_1$ die oben genannte Bedeutung haben, einer Acylierung mittels eines Säurechlorids der Formel (III)

ArCOCl (III),

in welcher Ar die oben angegebene Bedeutung hat, unterwirft, um ein Amid der Formel (XIII)

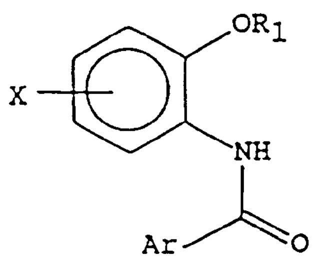

(XIII)

zu erhalten, das man mit 1-Brom-4-Chlorbutan umsetzt, worauf man die so erhaltene Verbindung der Formel (XIV)

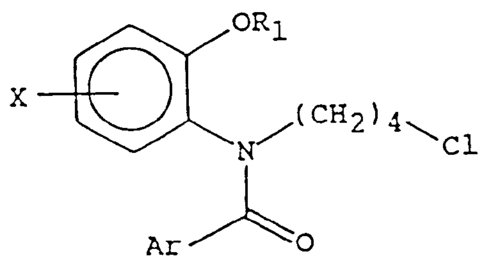

(XIV)

mit einem Amin der Formel (VII)

$HNR_2R_3$ (VII),

in welcher $R_2$ und $R_3$ die oben angegebene Bedeutung haben, zur Reaktion bringt.

## Claims

**Claims for the following Contracting States : AT, BE, CH, DE, FR, GB, IT, LI, LU, NL, SE.**

1. Compounds of general formula (I)

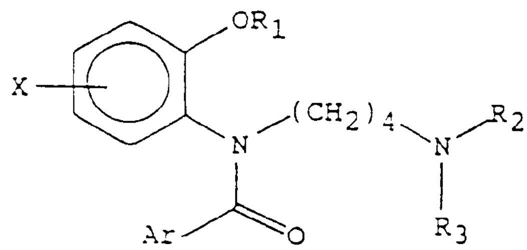

(I)

in which

X denotes a hydrogen or halogen atom or a trifluoromethyl, $C_1$-$C_4$ alkyl or $C_1$-$C_4$ alkoxy group,

$R_1$ denotes a linear or branched $C_2$-$C_8$ alkyl group, a $C_3$-$C_5$ cycloalkyl group or a cycloalkylmethyl group in which the ring is a $C_3$-$C_5$ ring,

$R_2$, taken separately, denotes a hydrogen atom or a $C_1$-$C_4$ alkyl group,

$R_3$, taken separately, denotes a hydrogen atom or a $C_1$-$C_4$ alkyl group, an optionally substituted phenylalkyl group or a pyridylalkyl group, or alternatively,

$R_2$ and $R_3$, taken together, denote, with the nitrogen atom to which they are attached, a pyrrolidinyl, piperidyl, morpholinyl, perhydrothiazinyl, piperazinyl or 4-methylpiperazinyl ring, and

Ar denotes

- either a phenyl group optionally bearing from one to three substituents chosen from halogen atoms and $C_1$-$C_4$ alkyl, $C_1$-$C_4$ alkoxy, methylenedioxy, trifluoromethyl, nitro and cyano groups,
- or a naphthyl, pyridyl, quinolinyl or isoquinolinyl group,

as well as their addition salts with pharmacologically acceptable acids.

2. Compounds according to Claim 1, characterised in that $R_1$ denotes an isobutyl group and Ar denotes a substituted phenyl group.

3. Compounds according to Claim 2, characterised in that X denotes a hydrogen atom and $R_2$ denotes a methyl group.

4. Compound according to Claim 3, characterised in that $R_3$ denotes a methyl group and Ar denotes a 2,3-dimethylphenyl group.

5. Compound according to Claim 3, characterised in that $R_3$ denotes a methyl group and Ar denotes a 3-trifluoromethylphenyl group.

6. Compound according to Claim 3, characterised in that $R_3$ denotes a methyl group and Ar denotes a 3-nitrophenyl group.

7. Compound according to Claim 3, characterised in that $R_3$ denotes a methyl group and Ar denotes a 2-methoxy-5-nitrophenyl group.

8. Compound according to Claim 3, characterised in that $R_3$ denotes a 2-(3-methoxyphenyl)ethyl group and Ar denotes a 3-trifluoromethylphenyl group.

9. Compound according to Claim 3, characterised in that $R_3$ denotes a 2-(3,4-dimethoxyphenyl)ethyl group and Ar denotes a 3-trifluoromethylphenyl group.

10. Compound according to Claim 3, characterised in that $R_3$ denotes a 2-(3-pyridyl)ethyl group and Ar denotes a 3-trifluoromethylphenyl group.

11. Compound according to Claim 3, characterised in that $R_3$ denotes a 2-(3,4-dimethoxyphenyl)ethyl group and Ar denotes a 2-methyl-3-nitrophenyl group.

12. Process for preparing the compounds according to Claim 1, characterised in that a diamine of formula (II)

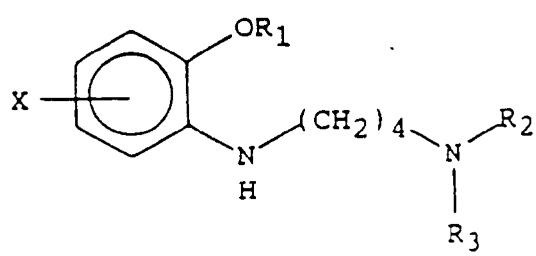

(II)

in which X, $R_1$, $R_2$ and $R_3$ are as defined in Claim 1, is subjected to an acylation by means of an acid chloride

of formula (III)

ArCOCl (III)

in which Ar is as defined in Claim 1.

13. Process for preparing the compounds according to Claim 1, characterised in that a benzenamine of formula (IV)

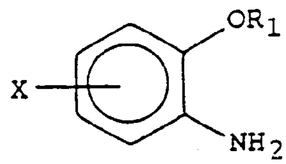

(IV)

in which X and $R_1$ are as defined in Claim 1, is subjected to an acylation by means of an acid chloride of the formula (III)

ArCOCl (III)

in which Ar is as defined in Claim 1, to obtain an amide of formula (XIII)

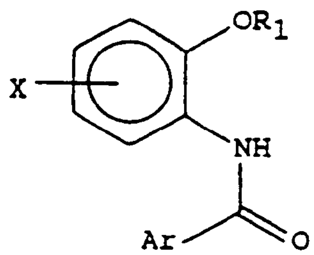

(XIII)

which is reacted with 1-bromo-4-chlorobutane, and the compound of formula (XIV) thereby obtained

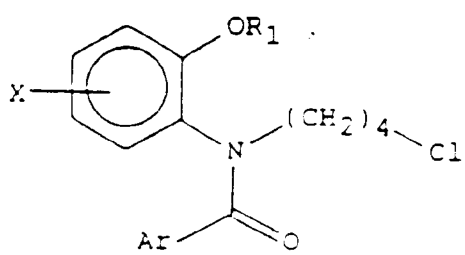

(XIV)

is then treated with an amine of formula (VII)

$HNR_2R_3$ (VII)

in which $R_2$ and $R_3$ are as defined in Claim 1.

14. Pharmaceutical composition, characterised in that it contains a compound according to one of Claims 1 to 11, in combination with a pharmaceutical excipient.

**Claims for the following Contracting States : ES, GR**

1. Process for preparing the compounds of general formula (I)

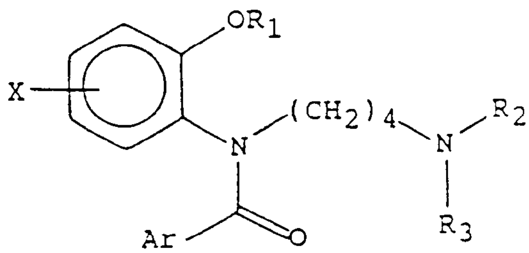

(I)

in which
X denotes a hydrogen or halogen atom or a trifluoromethyl, $C_1$-$C_4$ alkyl or $C_1$-$C_4$ alkoxy group,
$R_1$ denotes a linear or branched $C_2$-$C_8$ alkyl group, a $C_3$-$C_5$ cycloalkyl group or a cycloalkylmethyl group in which the ring is a $C_3$-$C_5$ ring,
$R_2$, taken separately, denotes a hydrogen atom or a $C_1$-$C_4$ alkyl group,
$R_3$, taken separately, denotes a hydrogen atom or a $C_1$-$C_4$ alkyl group, an optionally substituted phenylalkyl group or a pyridylalkyl group, or alternatively,
$R_2$ and $R_3$, taken together, denote, with the nitrogen atom to which they are attached, a pyrrolidinyl, piperidyl, morpholinyl, perhydrothiazinyl, piperazinyl or 4-methylpiperazinyl ring, and
Ar denotes

- either a phenyl group optionally bearing from one to three substituents chosen from halogen atoms and $C_1$-$C_4$ alkyl, $C_1$-$C_4$ alkoxy, methylenedioxy, trifluoromethyl, nitro and cyano groups,
- or a naphthyl, pyridyl, quinolinyl or isoquinolinyl group,

which process is characterised in that a diamine of formula (II)

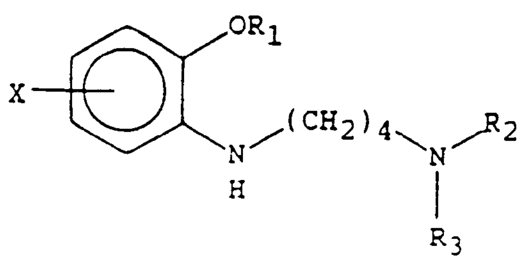

(II)

in which X, $R_1$, $R_2$ and $R_3$ are as defined above, is subjected to an acylation by means of an acid chloride of the formula (III)

ArCOCl (III)

in which Ar is as defined above.

2. Process for preparing the compounds of general formula (I)

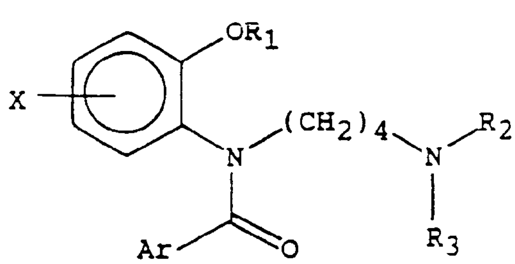

(I)

in which
X denotes a hydrogen or halogen atom or a trifluoromethyl, $C_1$-$C_4$ alkyl or $C_1$-$C_4$ alkoxy group,
$R_1$ denotes a linear or branched $C_2$-$C_8$ alkyl group, a $C_3$-$C_5$ cycloalkyl group or a cycloalkylmethyl group in which the ring is a $C_3$-$C_5$ ring,
$R_2$, taken separately, denotes a hydrogen atom or a $C_1$-$C_4$ alkyl group,
$R_3$, taken separately, denotes a hydrogen atom or a $C_1$-$C_4$ alkyl group, an optionally substituted phenylalkyl group or a pyridylalkyl group, or alternatively,

$R_2$ and $R_3$, taken together, denote, with the nitrogen atom to which they are attached, a pyrrolidinyl, piperidyl, morpholinyl, perhydrothiazinyl, piperazinyl or 4-methylpiperazinyl ring, and
Ar denotes

- either a phenyl group optionally bearing from one to three substituents chosen from halogen atoms and $C_1$-$C_4$ alkyl, $C_1$-$C_4$ alkoxy, trifluoromethyl, nitro and cyano groups,
- or a naphthyl, pyridyl, quinolinyl or isoquinolinyl group,

which process is characterised in that a benzenamine of formula (IV)

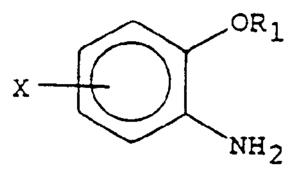

(IV)

in which X and $R_1$ are as defined above, is subjected to an acylation by means of an acid chloride of formula (III)

ArCOCl (III)

in which Ar is as defined above, to obtain an amide of formula (XIII)

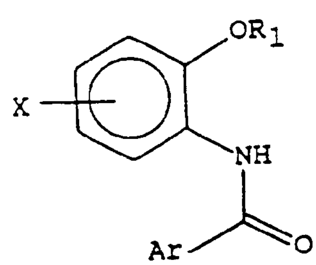

(XIII)

which is reacted with 1-bromo-4-chlorobutane, and the compound of formula (XIV) thereby obtained

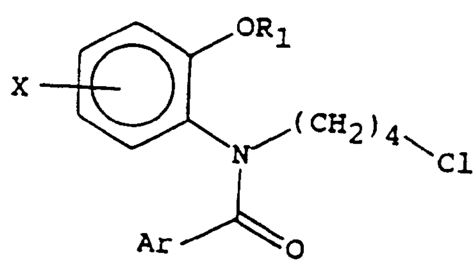

(XIV)

is treated with an amine of formula (VII)

$HNR_2R_3$ (VII)

in which $R_2$ and $R_3$ are as defined above.